# EUROPEAN PATENT APPLICATION

(11) **EP 3 718 514 A1**
(43) Date of publication of application: **07.10.2020**
(21) Application number: 18883754.6
(22) Date of filing: 29.11.2018
(51) Int. Cl.: A61F 2/66, C08K 3/04, C08K 3/22, C08K 3/36, C08L 7/00, C08L 9/00

(54) **RUBBER COMPOSITION FOR PROSTHETIC FOOT SOLE AND PROSTHETIC FOOT SOLE**

(30) Priority: 29.11.2017 JP 2017228651
(71) Applicant: Bridgestone Corporation, Tokyo 104-8340 (JP)
(72) Inventor: SAKURAI, Hideyuki, Tokyo 104-8340 (JP); NAKANE, Shinsuke, Tokyo 104-8340 (JP); ODAIRA, Miho, Tokyo 104-8340 (JP)
(74) Representative: Oxley, Robin John George
(86) International application number: PCT/JP2018/044091
(87) International publication number: WO 2019/107519

(57) **Abstract**

The present invention provides a rubber composition for a prosthetic foot sole exhibiting high grip performance in all weather conditions and excellent in durability, and the prosthetic foot sole. The rubber composition for a prosthetic foot sole and the prosthetic foot sole, include at least one rubber component selected from a natural rubber and a synthetic diene rubber and at least one filler selected from carbon black and an inorganic filler, wherein the total amount of the filler is 50 parts by mass or more and 150 parts by mass or less.

## Description

### Technical Field

The present invention relates to a prosthetic foot sole, and more particularly to a rubber composition for a prosthetic foot sole and a prosthetic foot sole.

### Background Art

People wearing a prosthetic foot desire a prosthetic foot that enables walking and running without anxiety. A prosthetic foot having a leaf spring is known (see, for example, PTL 1 and PTL 2). However, in wearing a prosthetic foot having a leaf spring, grip performance of the leaf spring itself is low, and therefore, a part of a commercially available shoe is diverted and used or a commercially available rubber sheet is cut and stuck to secure biting property (grip property) to a road surface.

### Citation List

### Patent Literature

PTL 1: JP 2017-35324 A
PTL 2: JP 2017-515525 T

### Summary of Invention

### Technical Problem

In prosthetic foot applications where the load applied to the sole is large, it is necessary to exhibit sufficient grip performance, and in particular, it is necessary to exhibit sufficient grip performance on a road surface wet by rain or the like (wet environment).

Accordingly, an object of the present invention is to provide a rubber composition for a prosthetic foot sole and a prosthetic foot sole which exhibit high grip performance in all weather conditions and are excellent in durability.

### Solution to Problem

The present inventors have found that a rubber composition for a prosthesis sole and a prosthesis sole which exhibit high grip performance in all weather conditions and are excellent in durability are obtained by incorporating a specific filler, and have completed the present invention.

The present invention provides the following [1] to [6].
[1] A rubber composition for a prosthetic foot sole, containing: at least one rubber component selected from a natural rubber and a synthetic diene rubber; and at least one filler selected from carbon black and an inorganic filler, wherein the total amount of the filler is 50 parts by mass or more and 150 parts by mass or less with respect to 100 parts by mass of the rubber component.
[2] The rubber composition for a prosthetic foot sole according to [1], wherein the filler contains the carbon black in an amount of 0 parts by mass or more and 90 parts by mass or less and the inorganic filler in an amount of 10 parts by mass or more and 100 parts by mass or less.
[3] The rubber composition for a prosthetic foot sole according to [1] or [2], wherein the inorganic filler is selected from at least one of silica and aluminum hydroxide.
[4] The rubber composition for a prosthetic foot sole according to any one of [1] to [3], wherein the rubber component contains at least one member selected from a natural rubber and a styrene-butadiene copolymer rubber.
[5] The rubber composition for a prosthetic foot sole according to any one of [1] to [4], wherein the carbon black has a nitrogen adsorption specific surface area of 30 m²/g or more and 200 m²/g or less.
[6] A prosthetic foot sole containing the rubber composition for a prosthetic foot sole according to any one of [1] to [5].

### Advantageous Effects of Invention

According to the present invention, it is possible to provide the rubber composition for a prosthetic foot sole and the prosthetic foot sole which exhibit the high grip performance in all weather conditions and are excellent in the durability.

### Brief Description of Drawings

Fig. 1 is a schematic view (part 1) for explaining a prosthetic foot using a rubber composition for a prosthetic foot sole according to an embodiment of the present invention.
Fig. 2 is a schematic view for explaining a prosthetic foot sole using a rubber composition for a prosthetic foot sole according to an embodiment of the present invention.
Fig. 3 is a schematic view (part 2) for explaining a prosthetic foot using a rubber composition for a prosthetic foot sole according to an embodiment of the present invention.

### Description of Embodiments

### [Rubber Composition for Prosthetic Foot Sole]

The rubber composition for a prosthetic foot sole according to an embodiment of the present invention will be described in detail.

A rubber composition for a prosthetic foot sole according to an embodiment of the present invention includes at least one rubber component selected from a natural rubber and a synthetic diene rubber, and at least one filler selected from carbon black and an inorganic filler, wherein the total amount of the filler is 50 parts by mass or more and 150 parts by mass or less with respect to 100 parts by mass of the rubber component.

Of the dynamic storage elastic modulus of the rubber composition for a prosthetic foot sole, the low-temperature assumed dynamic storage elastic modulus (E₁'), which is an average value at 5°C to 20°C, is preferably 5.0 MPa or more and 80.0 MPa or less, more preferably 20.0 MPa or more and 65.0 MPa or less, and still more preferably 40.0 MPa or more and 50.0 MPa or less from the viewpoint of highly expressing the abrasion resistance at a low temperature (5°C to 20°C), the dry grip performance, and the wet grip performance. When the value of the low-temperature assumed dynamic storage elastic modulus (E₁') is too small, the abrasion resistance at a low temperature (5°C to 20°C) is deteriorated, and when the value is too large, the grip performance at a low temperature (5°C to 20°C) is deteriorated. The reason for defining the average value from 5°C to 20°C is to evaluate the performance without being affected by the outside air temperature or the road surface temperature.

Of the dynamic storage elastic modulus of the rubber composition for a prosthetic foot sole, the high-temperature assumed dynamic storage elastic modulus (E₂'), which is an average value at 20°C to 50°C, is preferably 5.0 MPa or more and 60.0 MPa or less, more preferably 10.0 MPa or more and 45.0 MPa or less, and still more preferably 20.0 MPa or more and 30.0 MPa or less from the viewpoint of highly expressing the abrasion resistance at a high temperature (20°C to 50°C), the dry grip performance, and the wet grip performance. When the value of the high-temperature assumed dynamic storage elastic modulus (E₂') is too small, the abrasion resistance at a high temperature (20°C to 50°C) is deteriorated, and when the value is too large, the grip performance at a high temperature (20°C to 50°) is deteriorated. The reason for defining the average value from 20°C to 50°C is to evaluate the performance without being affected by the outside air temperature or the road surface temperature.

The rubber hardness (Hd) of the rubber composition for a prosthetic foot sole is measured in accordance with JIS K6253-3 (type A), and is preferably 50 degrees or more and 70 degrees or less, more preferably 52 degrees or more and 68 degrees or less, and still more preferably 54 degrees or more and 66 degrees or less from the viewpoint of improving the durability.

The tensile stress under 300% elongation (Md300) of the rubber composition for a prosthetic foot sole is measured in accordance with JIS K6251, and is preferably 8.0 MPa or more and 15.0 MPa or less, more preferably 10.5 MPa or more and 14.5 MPa or less, and still more preferably 11.0 MPa or more and 14.0 MPa or less from the viewpoint of improving the durability.

### (Rubber Component)

Examples of the rubber component include a natural rubber (NR) and a synthetic diene rubber. Specific examples of the synthetic diene rubber include a polybutadiene rubber (BR), a synthetic polyisoprene rubber (IR), a butyl rubber (IIR), a brominated butyl rubber (Br-IIR), a chlorinated butyl rubber (Cr-IIR), a styrene-butadiene copolymer rubber (SBR), and a styrene-isoprene copolymer rubber (SIR). The rubber component may be used alone or in combination of two or more kinds thereof. The rubber component may be modified or unmodified.

The rubber component preferably contains a styrene-butadiene copolymer rubber (SBR), and more preferably contains only a styrene-butadiene copolymer rubber (SBR) from the viewpoint of improving the grip performance on a wet road surface (wet grip performance) and the grip performance on a dry road surface (dry grip performance).

### (Filler)

Examples of the filler include carbon black and an inorganic filler. As the filler, one kind of carbon black or one kind of inorganic filler may be used. The filler is preferably used in combination of the carbon black and the inorganic filler from the viewpoint of exhibiting the respective effects of the carbon black and the inorganic filler.

In the description herein, the carbon black is not included in the inorganic filler.

The total amount of the mixing amount of the filler is preferably 50 parts by mass or more, more preferably 60 parts by mass or more, and still more preferably 70 parts by mass or more, with respect to 100 parts by mass of the rubber component from the viewpoints of improving the grip performance on a wet road surface (wet grip performance) and the grip performance on a dry road surface (dry grip performance) and improving the durability. The mixing amount of the filler is preferably 150 parts by mass or less, more preferably 130 parts by mass or less, and still more preferably 120 parts by mass or less with respect to 100 parts by mass of the rubber component from the viewpoint of the dispersibility.

### <Carbon Black>

By mixing carbon black as a filler, high elasticity can be obtained and the abrasion resistance can be improved.

The carbon black is not particularly limited, and for example, high, medium or low structure SAF, ISAF, IISAF, N339, HAF, HAF-HS, FEF, GPF or SRF grade carbon black, particularly SAF, ISAF, IISAF, HAF, HAF-HS or FEF grade carbon black is preferably used. The carbon black may be used alone or in combination of two or more kinds thereof.

As the carbon black, for example, commercially available products such as trade names "Seast 9" and "Seast 3H" manufactured by Tokai Carbon Co., Ltd. can be used.

The nitrogen adsorption specific surface area (N₂SA) of the carbon black is measured in accordance with JIS K 6217-2:2001, and is preferably 30 m²/g or more and 200 m²/g or less, more preferably 40 m²/g or more and 180 m²/g or less, and still more preferably 60 m²/g or more and 160 m²/g or less.

The mixing amount of the carbon black is preferably 1 part by mass or more, more preferably 3 parts by mass or more, and still more preferably 5 parts by mass or more with respect to 100 parts by mass of the rubber component from the viewpoint of improving the reinforcing property and the abrasion resistance of the rubber. Further, the mixing amount of the carbon black is preferably 90 parts by mass or less, more preferably 85 parts by mass or less, and still more preferably 80 parts by mass or less with respect to 100 parts by mass of the rubber component from the viewpoint of the dispersibility. However, in preparing a rubber exhibiting any color other than black color, the amount of the carbon black may be 0 parts by mass.

### <Inorganic Filler>

By mixing an inorganic filler as a filler, the grip performance (wet grip performance) and abrasion resistance on a wet road surface can be improved.

Examples of the inorganic filler include silica and aluminum hydroxide (Al(OH)₃). The inorganic filler is preferably selected from at least one of silica and aluminum hydroxide.

The mixing amount of the inorganic filler is preferably 10 parts by mass or more, more preferably 15 parts by mass or more, and still more preferably 20 parts by mass or more with respect to 100 parts by mass of the rubber component from the viewpoint of improving the grip performance (wet grip performance) and abrasion resistance on a wet road surface. The mixing amount of the inorganic filler is preferably 95 parts by mass or less, more preferably 90 parts by mass or less, and still more preferably 85 parts by mass or less with respect to 100 parts by mass of the rubber component from the viewpoint of the dispersibility.

### (Silica)

By mixing silica as an inorganic filler, the grip performance and abrasion resistance on a wet road surface can be improved.

The silica is not particularly limited, and examples thereof include wet method silica (hydrous silicic acid), dry method silica (anhydrous silicic acid), calcium silicate, and aluminum silicate, and among these, wet method silica is preferable. The silica may be used alone or in combination of two or more kinds thereof.

Examples of the silica include a trade name "Nipsil AQ" (BET specific surface area = 205 m²/g) manufactured by Tosoh Silica Corporation and a trade name "Ultrasil VN-3" (BET specific surface area = 175 m²/g) manufactured by Degussa Corporation.

The BET specific surface area of the silica is measured in accordance with ISO 5794/1, and is preferably in the range of 40 m²/g or more and 350 m²/g or less, more preferably in the range of 80 m²/g or more and 350 m²/g or less, and still more preferably in the range of 120 m²/g or more and 350 m²/g or less. When the BET specific surface area of the silica is in the above range, both the rubber reinforcing property and the dispersibility in the diene rubber can be achieved.

The mixing amount of the silica is preferably 40 parts by mass or more, more preferably 45 parts by mass or more, and still more preferably 50 parts by mass or more with respect to 100 parts by mass of the rubber component from the viewpoint of improving the grip performance (wet grip performance) and abrasion resistance on a wet road surface. The mixing amount of the silica is preferably 100 parts by mass or less, more preferably 95 parts by mass or less, and still more preferably 90 parts by mass or less with respect to 100 parts by mass of the rubber component from the viewpoints of the abrasion resistance and the dispersibility,

### (Aluminum Hydroxide)

By mixing aluminum hydroxide as an inorganic filler, the grip performance (wet grip performance) on a wet road surface can be improved.

The aluminum hydroxide is not particularly limited, and any of gibbsite and bayerite can be used.

Examples of the aluminum hydroxide include trade names "Hygilite H-43M" (average particle diameter: 0.75 pm), "Hygilite H-43" (average particle diameter: 0.75 pm), "Hygilite H-42M" (average particle diameter: 1.1 pm), "Hygilite H-42" (average particle diameter: 1.1 pm), "Hygilite H-32" (average particle diameter: 8 pm), "Hygilite H-31" (average particle diameter: 20 pm), "Hygilite H-21" (average particle diameter: 26 pm), "Hygilite H-10" (average particle diameter: 55 pm), and "Hygilite H-10A" (average particle diameter: 50 pm), all of which are manufactured by Showa Denko K.K. In addition, examples of the aluminum hydroxide include trade names "B1403" (average particle diameter: 1 pm), "B703" (average particle diameter: 2 pm), "B103" (average particle diameter: 8 pm), "B153" (average particle diameter: 15 pm), "B303" (average particle diameter: 30 pm), and "B53" (average particle diameter: 50 pm), all of which are manufactured by Nippon Light Metal Company, Ltd. Further, examples of the aluminum hydroxide include "C301" (average particle diameter: 2 pm), "C303" (average particle diameter: 3 pm), and "C308" (average particle diameter: 8 pm) manufactured by Sumitomo Chemical Co., Ltd.

The mixing amount of the aluminum hydroxide is preferably 1 part by mass or more, more preferably 5 parts by mass or more, and still more preferably 15 parts by mass or more with respect to 100 parts by mass of the rubber component from the viewpoint of improving the grip performance (wet grip performance) on a wet road surface. The mixing amount of the aluminum hydroxide is preferably 40 parts by mass or less, more preferably 35 parts by mass or less, and still more preferably 30 parts by mass or less with respect to 100 parts by mass of the rubber component from the viewpoint of the dispersibility.

### <Other Compounding Agents>

The rubber composition for a prosthetic foot sole according to the present invention can be mixed by appropriately selecting compounding agents (excluding fillers) usually used in the rubber industry. Examples of such compounding agents include an aging inhibitor, a softening agent (oil), a wax, a silane coupling agent, a vulcanization accelerator such as stearic acid, a vulcanization accelerator aid such as zinc oxide, and a vulcanizing agent such as sulfur. As the compounding agent, a commercially available product can be suitably used.

The mixing amount of the softening agent (oil) is preferably 100 parts by mass or less, more preferably 10 parts by mass or more and 75 parts by mass or less, and still more preferably 20 parts by mass or more and 50 parts by mass or less with respect to 100 parts by mass of the rubber component from the viewpoint of improving the abrasion properties. When the mixing amount of the softening agent (oil) is 100 parts by mass or less, the wet grip performance and the dry grip performance can be prevented from being deteriorated.

When the rubber component contains a styrene-butadiene copolymer rubber (SBR), an aromatic oil is preferably used as the softening agent (oil) from the viewpoint of compatibility with the styrene-butadiene copolymer rubber (SBR). As the softening agent (oil), it is preferable to use a naphthenic oil, a paraffinic oil or the like from the viewpoint of placing importance on the abrasion resistance at a low temperature (5°C to 20°C).

### <Production Method of Rubber Composition for Prosthetic Foot Sole>

The rubber composition for a prosthetic foot sole of the present invention is obtained by kneading the aforementioned components. The kneading method may be carried out according to a method commonly practiced by those skilled in the art, and for example, all the components other than sulfur, a vulcanization accelerator and zinc oxide (in the case of using a vulcanization retarder, the vulcanization retarder is further included) are kneaded at 100°C to 200°C using a Banbury mixer, a Brabender, a kneader, a high shear mixer or the like, and then sulfur, the vulcanization accelerator and zinc oxide (and further the vulcanization retarder, if necessary) are added and kneaded at 60°C to 130°C using a kneading roll machine or the like.

### [Prosthetic Foot Sole]

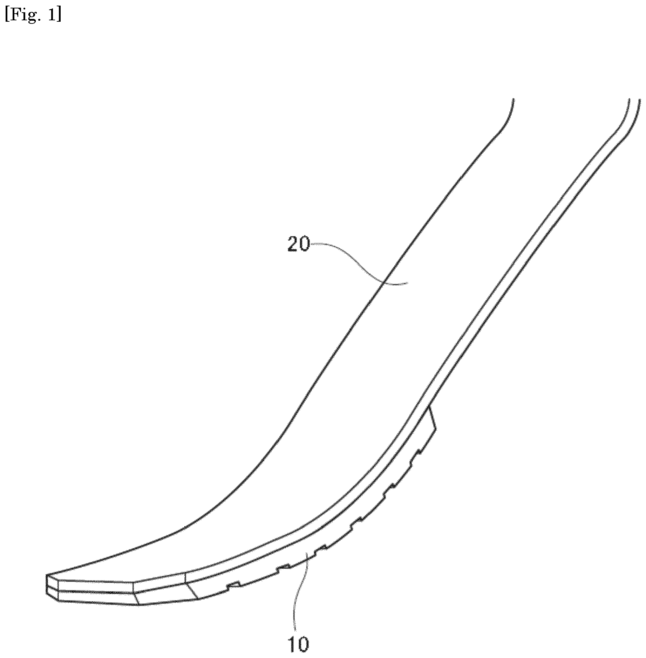

As shown in Fig. 1, a prosthetic foot sole 10 according to an embodiment of the present invention is provided at a position where a prosthetic foot 20 comes into contact with a road surface.

The means for attaching the prosthetic foot sole 10 to the prosthetic foot 20 is not particularly limited, and for example, a fixing means using an adhesive or a fastening means using a fastener may be employed. When the fastening means is employed, replacement of the prosthetic foot sole 10 is facilitated. Examples of the fastener used in the fastening means include a screw, a belt, a string, a fastener, and a buckle.

The prosthetic foot sole 10 according to the embodiment of the present invention is formed of the above-described rubber composition for a prosthetic foot sole. The method of forming the prosthetic foot sole 10 is not particularly limited, and it can be formed by a conventionally known method.

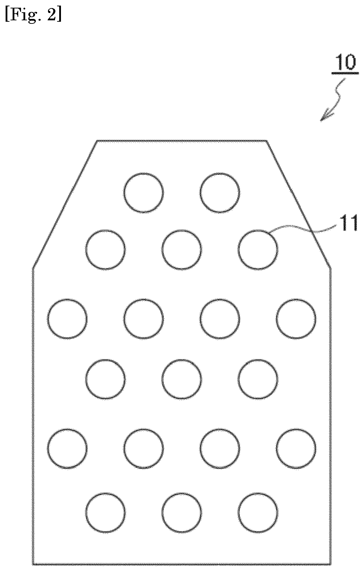

As shown in Fig. 2, the prosthetic foot sole 10 is provided with a sole pattern 11, whereby drainage function can be improved and the grip performance (wet grip performance) on a wet road surface can be improved. The sole pattern 11 is not particularly limited, and examples thereof include a circular pattern, a rectangular pattern, a polygonal pattern, and a Bishamon kikko pattern (based on the pattern combining three hexagons). The sole pattern 11 may be a single pattern or a combination of two or more patterns. As the sole pattern 11, a pattern of a single size may be used, or patterns of 2 or more sizes may be used in combination. A plurality of the sole patterns 11 are preferably arranged on the sole, and more preferably arranged on the entire surface of the sole from the viewpoints of improving the wet grip performance and improving the cushioning property (flexibility).

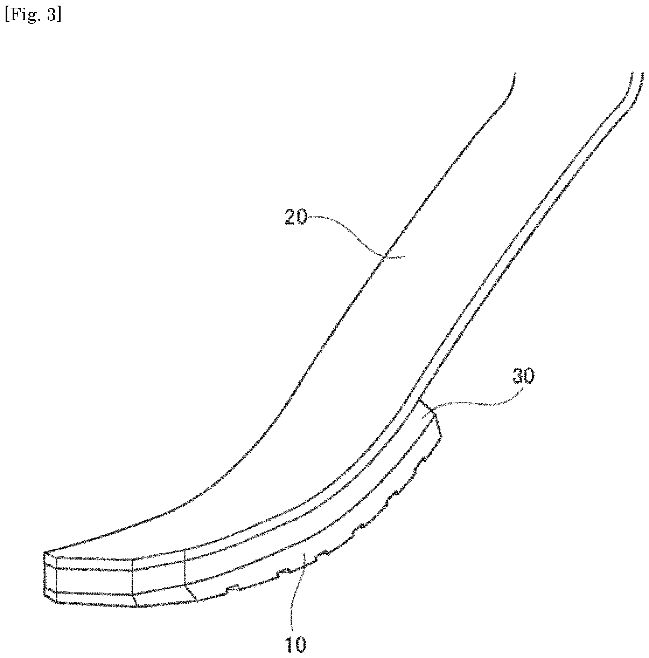

As shown in Fig. 3, the prosthetic foot sole 10 according to the embodiment of the present invention is preferably provided with an interference layer 30 between the prosthetic foot sole 10 and the prosthetic foot 20. The interference layer 30 can interfere with the impact from the road surface and improve the cushioning property (flexibility).

The interference layer 30 is not particularly limited, and an elastic material such as a urethane resin material can be used.

### <Production Method of Prosthetic Foot Sole>

First, the above-described rubber composition for a prosthetic foot sole is processed into a rubber sheet by roll molding, calendar molding, or the like. Then, the obtained rubber sheet is molded by, for example, a mold as necessary, and vulcanized at a vulcanization temperature of 130°C or higher to obtain the prosthetic foot sole 10.

### Examples

Hereinafter, the present invention will be described in more detail with reference to examples, but the present invention is not limited to the following examples.

### <Dynamic Storage Elastic Modulus>

A sample having a length of 40 mm, a width of 5 mm, and a thickness of 2 mm was prepared. The dynamic storage elastic modulus (E') of this sample was measured using a spectrometer manufactured by Toyo Seiki Seisaku-sho Ltd. under the measurement conditions of an initial load of 160 g, a dynamic strain of 1%, and a frequency of 52 Hz from -45°C to 63°C at a rate of temperature increase of 3°C/min.

From the obtained data, a low-temperature assumed dynamic storage elastic modulus (E₁'), which is an average value from 5°C to 20°, is determined. In addition, a high-temperature assumed dynamic storage elastic modulus (E₂'), which is an average value from 20° to 40°C, was determined from the obtained data.

The lower the values of the low-temperature assumed dynamic storage elastic modulus (E₁') and the high-temperature assumed dynamic storage elastic modulus (E₂'), the better the cushioning property (flexibility).

### <Rubber Hardness>

The rubber hardness (Hd) was measured in accordance with JIS K6253-3 (type A).

### <Tensile Stress under 300% Elongation>

The tensile stress under 300% elongation (Md300) was measured in accordance with JIS K6251.

### <Wet Grip Performance>

For Examples 1 and 2, a frictional force generated when a vulcanized rubber having a long diameter of 40 mm, a short diameter of 20 mm, and a thickness of 2 mm was pressed against a fixed wet iron plate surface and reciprocated was detected by a load cell to calculate a dynamic friction coefficient. For Comparative Examples 1 to 3, the dynamic friction coefficient is calculated under the same conditions as in Examples 1 and 2. The measurement was performed at 15°C.

For each example, Comparative Example 1 was indexed as 100. The larger the index value, the better the wet grip performance.

### <Dry Grip Performance>

For Examples 1 and 2, a frictional force generated when a vulcanized rubber having a long diameter of 40 mm, a short diameter of 20 mm, and a thickness of 2 mm was pressed against a fixed dry concrete road surface and reciprocated was detected by a load cell to calculate a dynamic friction coefficient. For Comparative Examples 1 to 3, the dynamic friction coefficient is calculated under the same conditions as in Examples 1 and 2. The measurement was performed at room temperature.

For each example, Comparative Example 1 was indexed as 100. The larger the index value, the better the dry grip performance.

### <Abrasion Evaluation>

The amounts of abrasion of Examples 1 and 2 were measured using a DF tester manufactured by NIPPO LTD., and the amounts of abrasion were calculated. For Comparative Examples 1 to 3, the amounts of abrasion are calculated under the same conditions as those in Examples 1 and 2. The measurement is performed at 30°C.

For each example, Comparative Example 1 is indexed as 100. The smaller the index value, the better the abrasion resistance.

### [Examples 1 and 2]

In Examples 1 and 2, the rubber compositions for a prosthetic foot sole were prepared according to the mixing contents shown in Table 1. Thereafter, the mixture was vulcanized in a vulcanizer at 160°C for 15 minutes to obtain a vulcanized rubber.

The rubber compositions for a prosthetic foot sole of Examples 1 and 2 were evaluated as described above, and the results are shown in Table 1.

### [Comparative Examples 1 to 3]

In Comparative Examples 1 to 3, the rubber compositions for a prosthetic foot sole are prepared according to the mixing contents shown in Table 1. Thereafter, the mixture is vulcanized in a vulcanizer at 160°C for 15 minutes to obtain a vulcanized rubber.

The rubber compositions for a prosthetic foot sole of Comparative Examples 1 to 3 were evaluated as described above, and the results are shown in Table 1.

**Table 1**

| Mixing contents (parts by mass) | | Example 1 | Example 2 | Comparative Example 1 | Comparative Example 2 | Comparative Example 3 |
|---|---|---|---|---|---|---|
| Rubber component | NR | | | | | |
| | SBR*¹ | 134 | 134 | 134 | 134 | 134 |
| Filler | Carbon black*² | 5 | 80 | | | |
| | Carbon black*³ | | | 30 | | 80 |
| | Silica*⁴ | 75 | | | 5 | 100 |
| | Aluminum hydroxide*⁵ | | 20 | | | |
| Other compounding agents | Stearic acid | 1 | 1 | 1 | 1 | 1 |
| | Zinc oxide | 2 | 2 | 2 | 2 | 2 |
| | Wax*⁶ | 2 | 2 | 2 | 2 | 2 |
| | Aging inhibitor*⁷ | 1.7 | 1.7 | 1.7 | 1.7 | 1.7 |
| | Silane coupling agent*⁸ | 7.5 | | | 0.5 | 10 |
| Dynamic storage elastic modulus | Low-temperature assumed (E₁') (MPa) | 43.5 | 47.5 | 17.0 | 12.0 | 85.0 |
| | High-temperature assumed (E₂') (MPa) | 26.1 | 27.7 | 10.2 | 7.2 | 51.0 |
| Rubber hardness | Hd (degree) | 57.0 | 64.0 | 53.0 | 51.0 | 80.0 |
| Tensile stress under 300% elongation | Md300 (MPa) | 13.1 | 12.5 | 10.1 | 12.2 | 10.5 |
| Wet grip performance | (Index) | 204 | 284 | 100 | 86 | 110 |
| Dry grip performance | (Index) | 118 | 112 | 100 | 84 | 116 |
| Abrasion evaluation | (Index) | 18 | 23 | 100 | 160 | 38 |

| | | | | | | |
|---|---|---|---|---|---|---|
| [Note] *1: Styrene-butadiene copolymer rubber, manufactured by JSR Corporation, trade name "JSR 0150" (34.0 parts, oil-extended) *2: Trade name "Seast 9", SAF (N₂SA: 145 m²/g), manufactured by Tokai Carbon Co., Ltd. *3: Trade name "Seast 3H", HAF-HS (N₂SA: 80 m²/g), manufactured by Tokai Carbon Co., Ltd. *4: Trade name "Nipsil AQ" (BET surface 205 m²/g), manufactured by Tosoh Silica Corporation *5: "Hygilite H-43M" (average particle diameter: 0.75 pm), manufactured by Showa Denko K.K. *6: Microcrystalline wax, trade name "OZOACE-0280", manufactured by Nippon Seiro Co., Ltd. *7: N-(1,3-dimethylbutyl)-N'-phenyl-p-phenylenediamine, trade name "Nocrack 6C", manufactured by Ouchi Shinko Chemical Industries Co. Ltd. *8: Trade name "ABC-856", manufactured by Shin-Etsu Chemical Co., Ltd. | | | | | | |

From Table 1, the rubber compositions for a prosthetic foot sole of Examples 1 and 2 were good in each evaluation of the dynamic storage elastic modulus, the rubber hardness, the tensile stress under 300% elongation, the wet grip performance, the dry grip performance, and the abrasion evaluation.

### <Wet Grip Performance (Friction Coefficient)>

For Examples 3 and 4, a vulcanized rubber having a Bishamon kikko pattern was cut out so as to conform to a measuring jig having a long diameter of 40 mm and a short diameter of 20 mm, and a friction force generated when the vulcanized rubber was pressed against a fixed wet iron plate road surface and reciprocated was detected by a load cell to calculate a dynamic friction coefficient. For Comparative Example 4, a thermo-plastic polyurethane having a Bishamon kikko pattern was cut out so as to conform to a measuring jig having a long diameter of 40 mm and a short diameter of 20 mm, and a friction force generated when the thermo-plastic polyurethane was pressed against a fixed wet iron plate surface and reciprocated was detected by a load cell to calculate a dynamic friction coefficient. The measurement was performed at 15°C.

For each example, Comparative Example 4 is indexed as 100. The larger the index value, the better the wet grip performance.

### <Wet Grip Performance (Sensory Evaluation)>

The produced prosthetic foot sole was attached to the prosthesis foot of the prosthetic foot wearer, and the sensory evaluation was performed by running on a wet iron plate surface such as a manhole, a white line of a crosswalk, and a stone pavement. Those who did not feel anxiety of slipping were evaluated as "A", those who felt anxiety of slipping slightly were evaluated as "B", and those who felt fear of losing balance by slipping were evaluated as "C".

### [Example 3]

A prosthetic foot sole of Example 3 was produced using the rubber composition for a prosthetic foot sole of Example 1. In the prosthetic foot sole, a Bishamon kikko pattern is arranged on the entire surface of the sole as a sole pattern.

Table 2 shows the results of the above evaluations performed on the prosthetic foot sole of Example 3.

### [Example 4]

A prosthetic foot sole of Example 4 was produced using the rubber composition for a prosthetic foot sole of Example 2. In the prosthetic foot sole, a Bishamon kikko pattern is arranged on the entire surface of the sole as a sole pattern.

Table 2 shows the results of the above evaluations performed on the prosthetic foot sole of Example 4.

### [Comparative Example 4]

A commercially available shoe sole made of thermoplastic polyurethane and having a Bishamon kikko pattern was used as Comparative Example 4. In the prosthetic foot sole, a Bishamon kikko pattern is arranged on the entire surface of the sole as a sole pattern.

Table 2 shows the results of the above evaluations performed on the prosthetic foot sole of Comparative Example 4.

**Table 2**

| | | | Example 3 | Example 4 | Comparative Example 4 |
|---|---|---|---|---|---|
| Wet grip performance | Frictional coefficient µ | (Index) | 110 | 135 | 100 |
| | Sensory evaluation | | B | A | C |

From Table 2, the rubber compositions for a prosthetic foot sole of Examples 3 and 4 were good in each evaluation of the wet grip performance.

### Industrial Applicability

The rubber composition for a prosthetic foot sole of the present invention is suitably used as a sole material of a prosthetic foot, particularly as a sole material of a leaf spring-shaped prosthetic foot for competition.

### Reference Signs List

- 10:: Prosthetic foot sole
- 11:: Sole pattern
- 20:: Prosthetic foot
- 30:: Interference layer

## Claims

1. A rubber composition for a prosthetic foot sole, comprising: at least one rubber component selected from a natural rubber and a synthetic diene rubber; and at least one filler selected from carbon black and an inorganic filler, wherein the total amount of the filler is 50 parts by mass or more and 150 parts by mass or less with respect to 100 parts by mass of the rubber component.

2. The rubber composition for a prosthetic foot sole according to claim 1, wherein the filler contains the carbon black in an amount of 0 parts by mass or more and 90 parts by mass or less and the inorganic filler in an amount of 10 parts by mass or more and 100 parts by mass or less.

3. The rubber composition for a prosthetic foot sole according to claim 1 or 2, wherein the inorganic filler is selected from at least one of silica and aluminum hydroxide.

4. The rubber composition for a prosthetic foot sole according to any one of claims 1 to 3, wherein the rubber component contains at least one member selected from a natural rubber and a styrene-butadiene copolymer rubber.

5. The rubber composition for a prosthetic foot sole according to any one of claims 1 to 4, wherein the carbon black has a nitrogen adsorption specific surface area of 30 m²/g or more and 200 m²/g or less.

6. A prosthetic foot sole comprising the rubber composition for a prosthetic foot sole according to any one of claims 1 to 5.
